# EUROPEAN PATENT APPLICATION

(11) **EP 1 655 298 A1**
(43) Date of publication of application: **10.05.2006**
(21) Application number: 04026041.6
(22) Date of filing: 03.11.2004
(51) Int. Cl.: C07D 403/10, A61K 31/41

(54) **Novel polymorph forms of candesartan cilexetil**

(71) Applicant: LEK Pharmaceuticals d.d., 1526 Ljubljana (SI)
(72) Inventor: Antoncic, Ljubomir, 1000 Ljubljana (SI); Copar, Anton, 1275 Smartno pri Litiji (SI); Jeriha, Alenka, 1260 Ljubljana-Polje (SI)
(74) Representative: Dietz, Jörg-Reimar

(57) **Abstract**

New crystalline forms of candesartan cilexetil is prepared from the solution in a chlorinated solvent by addition of an alkane while new amorphous is prepared from above solution by precipitating by addition of cycloalkane. The form depends on the concentration of a solute in a chlorinated solvent.

## Description

### FIELD OF THE INVENTION

The present invention relates to the novel crystalline forms of candesartan cilexetil, to processes for their preparation and to pharmaceutical compositions containing them.

### BACKGROUND OF THE INVENTION

Candesartan cilexetil is an antihypertensive agent and its therapeutic uses were disclosed in US 5,196,444, which also disclosed a crystalline form of candesartan cilexetil. Two crystalline forms of candesartan cilexetil, form I and form II, are described in Chem. Pharm. Bull. 47.2), 182-186 (1999) as well as the amorphous substance obtained by grinding. The amorphous powders mentioned in US 5,196,444 are said to be unstable by heat and impractical in production, which is consitent with the fact that the melting point of the amorphous solid mechanically obtained could not be specified.

Dioxane solvate and additional two crystalline forms of candesartan cilexetil were described in WO 04085426.

Present invention discloses new crystalline forms of candesartan cilexetil and, processes for preparing these forms as well as novel process to prepare amorphous candesartan cilexetil and pharmaceutical compositions containing them.

### BRIEF DESCRIPTION OF THE DRAWINGS

Figure 1 is an x-ray powder diffraction pattern of candesartan cilexetil Form 5.
Figure 2 is an x-ray powder diffraction pattern of candesartan cilexetil Form 5 of another batch.
Figure 3 is an x-ray powder diffraction pattern of candesartan cilexetil Fomr 6.
Figure 4 is an x-ray powder diffraction pattern of amorphous candesartan cilexetil

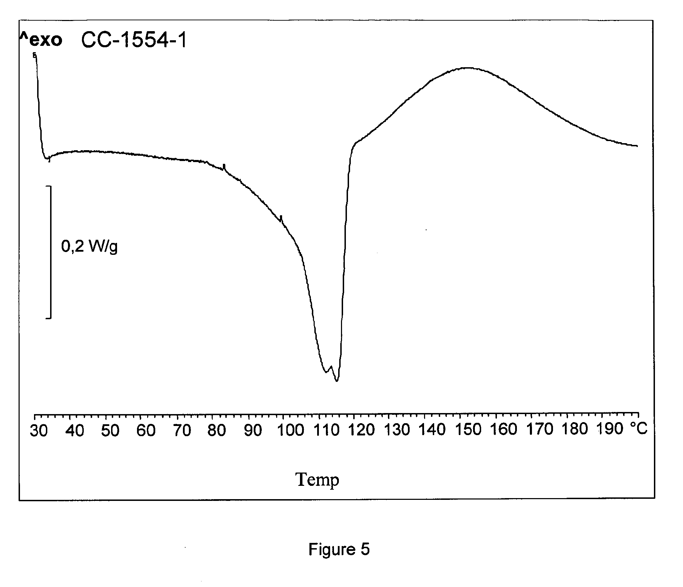

Figure 5 is a DSC thermogram of candesartan cilexetil Form 5.

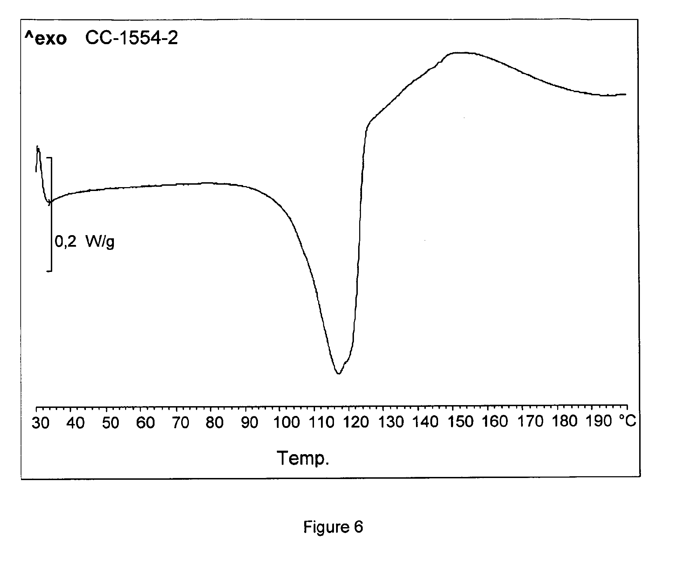

Figure 6 is a DSC thermogram of candesartan cilexetil Form 5 of another batch.

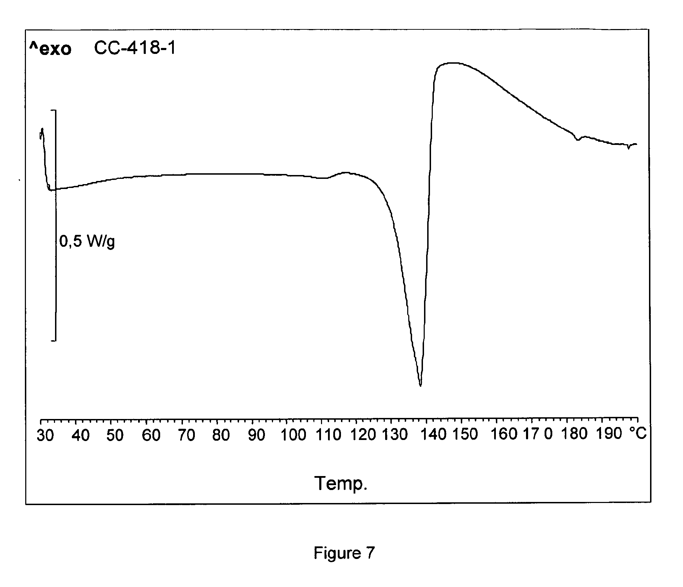

Figure 7 is a DSC thermogram of candesartan cilexetil Fomr 6.
Figure 8 is a DSC thermogram of amorphous candesartan cilexetil
Figure 9 is an x-ray powder diffraction pattern of candesartan cilexetil Form 7
Figure 10 is an x-ray powder diffraction pattern of candesartan cilexetil Form 8

### DESCRIPTION OF THE INVENTION

Polymorph forms of candesartan cilexetil can be obtained by a process which comprises: a) dissolving candesartan cilexetil, which may be obtained by the known methods, in chlorinated solvent; b) concentrating thus obtained solution and c) adding a hydrocarbon to the above concentrated solution; whereas the polymorph form produced depends on the concentration of aforesaid solution in a chlorinated solvent.

If the solution of candesartan cilexetil in a chlorinated solvent such as chloroform or dichloromethane is concentrated until concentration of about 0,26g to about 0,27g of candesartan cilexetil / g of the concentrate a Form 5, is obtained and if the aforesaid solution is concentrated until concentration of about 0,15g to about 0,21g or alternatively of about 0,5g to about 1,5g of candesartan cilexetil / g of the concentrate a Form 6 is obtained and if the aforesaid solution is concentrated until concentration of about 0,4 of candesartan cilexetil / g of the concentrate Form 8 is formed and by concentrating to about 0,55g - 0,58g of candesartan cilexetil / g of the concentrate Form 7 is formed, while in the range of 0,3g to about 0,4g or under certain conditions in the range of 0,3 g to 0,5 g of candesartan cilexetil / g of the concentrate amorphous candesartan cilexetil can be obtained by addition of cycloalkane.

**Table bellow shows dependence of polymorph formed of the concentration of candesartan cilexetil in a chlorinated solvent.**

| **Mass of solute per 1 g of solution** | **Polymorph** | **Typical X-ray diffraction peaks [° 2 Theta]** |
|---|---|---|
| 0,1 - 0,21 (preferred 0,15 - 0,21) | Form 6 | 6,9; 8,9; 16,6; 17,4; 19,4 |
| 0,22 - 0,3 (preferred 0,26 - 0,27) | Form 5 | 6,1; 11,6; 20,0; 21,2; 25,6 |
| 0,4 | Form 8 | 7,1; 16,3; 17,8; 19,5; 20,2; 24,0 |
| 0,55-0,58 | Form 7 | 6,0; 9,0; 12,0; 21,3; 22,4 |
| 0,6 - 2,0 (preferred 0,6-1,5) | Form 6 | 6,9; 8,9; 16,6; 17,4; 19,4 |

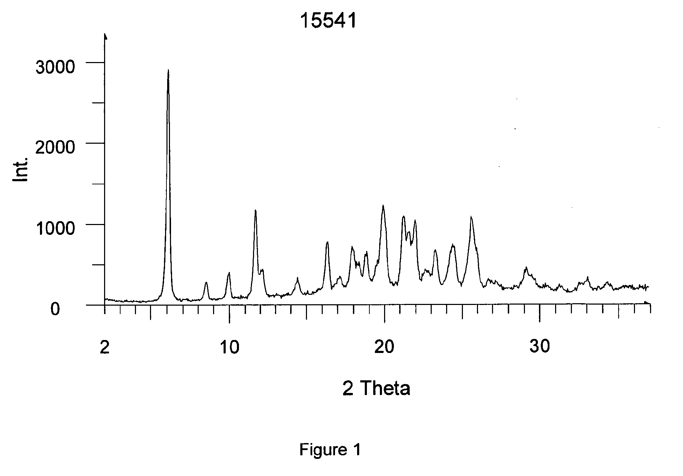

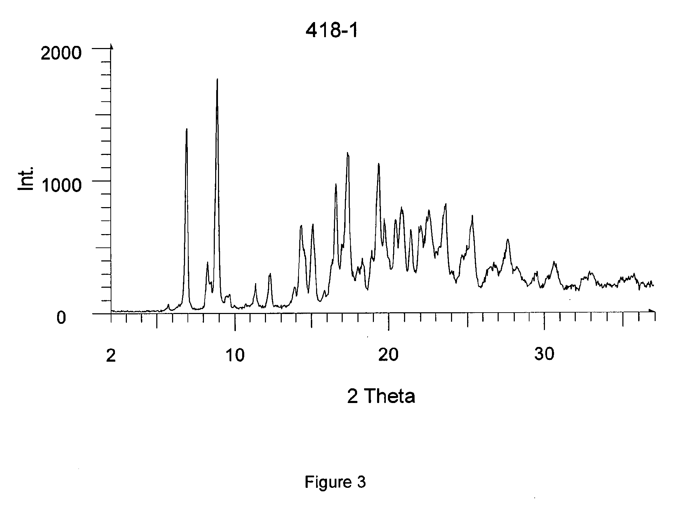

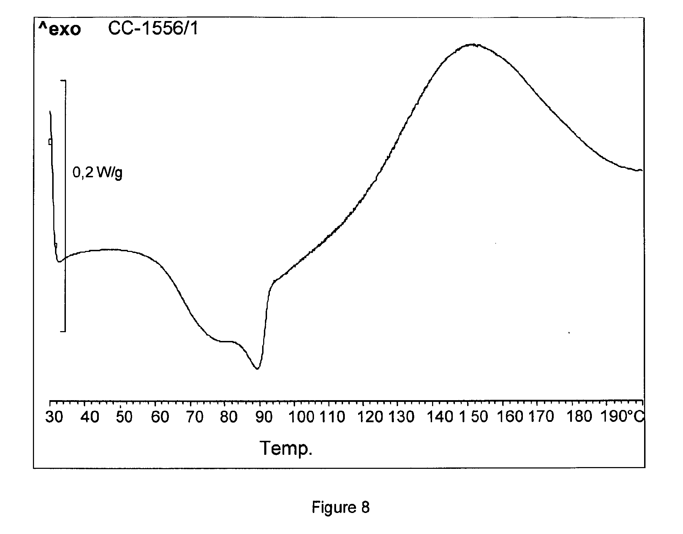

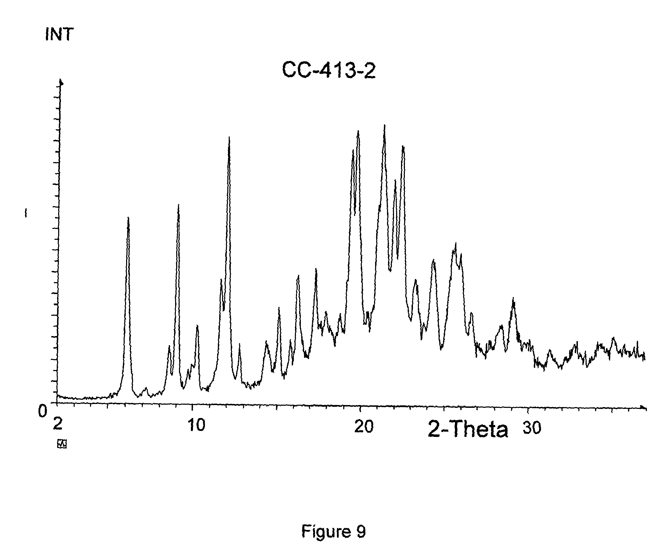

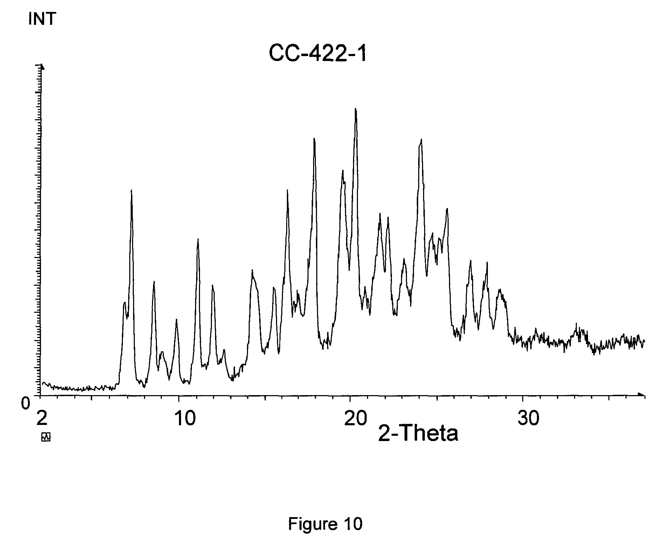

Above mentioned polymorph forms are characterized by their physical and chemical properties, for example Form 5 by an x-ray powder diffraction pattern such as in Figure 1 comprising peaks at about 11,6; 20,0; 21,2; 25,6 ± 0,2° 2Theta; or Form 6 by by an x-ray powder diffraction pattern such as in Figure 3 comprising peaks at about 6,9; 8,9; 16,6; 17,4; 19,4± 0,2° 2Theta; or Form 7 by by an x-ray powder diffraction pattern such as in Figure 9 comprising peaks at about 6,0; 9,0; 12,0; double peak between 19 and 20 (19,4 and 19,8); 21,3; 22,4; 25,6 ± 0,2° 2Theta; or Form 8 by an x-ray powder diffraction pattern such as in Figure 10 comprising peaks at about 7,1; 16,3; 17,8; 19,5; 20,2; 24,0 ± 0,2° 2Theta or an amorphous form exhibiting a continuum of X-ray diffractions throughout the entire difractogram scale and a thermogram as presented on Figure 8.

Present process allows advantages over previously known processes, where different polymorphs have been obtained from either lower alcohols, their mixture with water and a mixture of lower alkyl ketone with water or acetone or dioxane or toluene or methyl tert buthyl ether, at different temperatures while our process allows the unification of solvents at room temperature in order to produce various polymorph by essentially varying only the concentration of the solute.

The solubility studies of the novel polymorphs show faster dissolution at the physiologically important conditions. By our process we have been also able to produce form with substantially large surface area. Each of the polymorphs obtained exhibits a detectable melting point while the prior art processes using mechanical grinding has produced a heat unstable substance.

In one embodiment of our invention in case candesartan cilexetil, is dissolved in chlorinated solvent, preferably in dichloromethane or chloroform and the obtained solution is concentrated and thus obtained concentrate is precipitated with an hydrocarbon for example a liquid hydrocarbon such as alkane, preferably with hexane or its derivative, for example methylcyclohexane or n-hexane, a solid is formed which is a new crystalline form of candesartan cilexetil (Form 5)

Applying the above process, where however the obtained clear solution in chlorinated solvent is comparably less or more concentrated after the workup with methylcyclohexane or n-hexane a new crystalline form of candesartan cilexetil (Form 6) is formed.

At the intermediate concentration ranges additional polymorph forms of candesartan cilexetil can be obtained.

Contrary to the teaching of prior art where amorphous candesartan cilexetil was obtained mechanically from other crystalline forms, but no X-ray data on thus obtained substance was presented to confirm whether it is indeed amorphous, we have as yet another ambodiment of the invention developed a process in which candesartan cilexetil is dissolved in chlorinated solvent, preferably in dichloromethane or chloroform and the obtained solution is concentrated and thus obtained concentrate is precipitated with a liquid cyclic hydrocarbon, preferably with cyclohexane or cyclopentane an amorphous solid is formed that exhibits a continuum of X-ray diffractions throughout the entire difractogram scale. The amorphous form obtainable by this process exhibits a melting paint 106 - 109,4 °C whereas the previously mechanically obtained amorphous form was already liquid phase at 75 °C.

In accordance with the present invention, there is provided a pharmaceutical composition comprising amorphous candesartan cilexetil or Form 5 or Form 6 or Form 7 or Form 8 of candesartan cilexetil alone or in combination with another active ingredient such as hydrochlorotiazde and a pharmaceutically acceptable carrier comprising inactive ingredients such as fillers, binders, disintegrants, glidants, lubricants and other excipients.

The new polymorphs of candesartan cilexetil can be produced in substantially pure form or in a mixture. Depending on the desired characteristic, for example desired dissolution properties, the substantially pure polymorph forms can be incorporated into a pharmaceutical composition in form or alternatively a mixture thereof.

The described forms of candesartan cilexetil have a potent antihypertensive activity and incorporated pharmaceutical composition can be in a form suitable for peroral or parental application. Pharmaceutical composition in accordance with this invention can be embodied for example in form of tablet, capsules, pellets, granules and supozitories or their combined forms. Solid pharmaceutical compositions can be shielded, for example coated with the aim of increasing peletibility or regulating the disintegration or absorption.

The pharmaceutical compositions in accordance with our invention are useful as therapeutic agents for treating circulatory system diseases such as hypertensive diseases, heart diseases (e.g. hypercardia, heart failure, cardiac infarction, etc.), strokes, cerebral apoplexy, nephritis.

### DETAILED DESCRIPTION OF THE INVENTION

### A new crystalline form of candesartan cilexetil (Form 5)

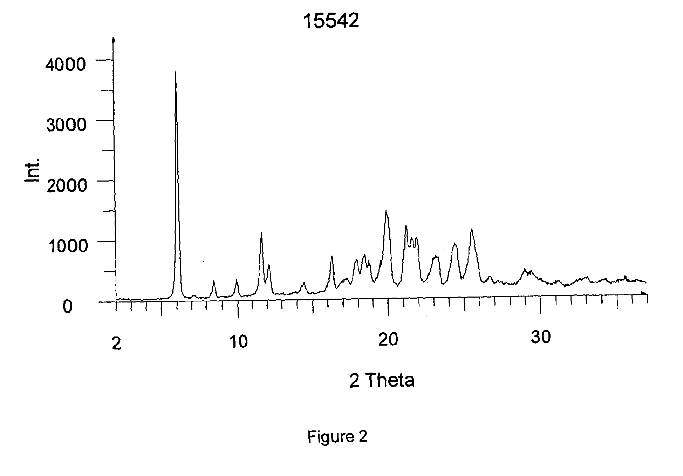

To obtain a Form 5 of candesartan cilexetil, candesartan cilexetil, which may be obtained by the known methods, is dissolved in chlorinated solvent, such as dichloromethane or chloroform in the concentration of 0,01 g to 0,35 g of candesartan cilexetil / g of the solvent at room temperature and the obtained solution is concentrated in vacuum to the concentration of 0,22 g - 0,3 g, preferably 0,26 g - 0,27g of candesartan cilexetil / g of the concentrate and thus obtained concentrate is precipitated by quick addition(in few seconds) of a liquid hydrocarbon, preferably n-heptane or hexane or its derivative, for example methylcyclohexane or n-hexane in the concentration 2 ml-50 ml of hydrocarbon/g of the concentrate at room temperature a solid is formed which is a new crystalline form of candesartan cilexetil (Form 5)
The candesartan cilexetil Form 5 is for example characterized by an x-ray powder diffraction pattern having peaks at about 4,5; 6,1; 7,2; 8,5; 9,9; 10,7; 11,7; 12,1; 13,0; 13,8; 14,4; 16,3; 17,1; 17,9; 18,3; 18,8; 19,5; 19,9; 21,2; 21,5; 22,0; 22,6; 22,8; 23,3; 24,4; 25,7; 26,0; 26,7; 27,2; 28,5; 29,2; 29,4; 30,5; 31,3; 32,6; 33,1; 34,3; 35,5 ± 0,2° 2Theta. Of those the most characteristic are the peaks at about 6,1; 11,7; 16,3; 17,9; 18,8; 19,9; 21,2; 21,5; 22,0; 23,3; 24,4; 25,7; 26,0° Figures 1 and 2 shows typical x-ray powder diffraction pattern of candesartan cilexetil Form 5 with strongest peaks at about 6,1 (the most intense); 11,6; 20,0; 21,2; 25,6 ± 0,2°. The candesartan cilexetil Form 5 is further characterized by DSC, IR and m.p.

### A new crystalline form of candesartan cilexetil (Form 6)

To obtain a Form 6 of candesartan cilexetil, candesartan cilexetil, which may be obtained by the known methods, is dissolved in chlorinated solvent, such as dichloromethane or chloroform in the concentration of 0,01 g -1,5 g of candesartan cilexetil /g of the solvent at room temperature and the obtained solution is concentrated in vacuum to the concentration of between about 0,1 g and 0,21 g, preferably 0,15 g - 0,21 g of candesartan cilexetil/g of the concentrate or between about 0,6 g to about 2 g, preferably 0,6 g - 1.5 g of candesartan cilexetil/g of concentrate alternatively and thus obtained concentrate is precipitated by quick addition (in few seconds) of a liquid cyclic hydrocarbon, preferably with methylcyclohexane or straight chain hydrocarbon or its derivative, for example n-heptane or n-hexane in the concentration of 2 ml - 40 ml of hydrocarbon/g of the concentrate at room temperature a solid is formed which is a new crystalline form of candesartan cilexetil (Form 6). The candesartan cilexetil Form 6 is for example characterized by an x-ray powder diffraction pattern having peaks at about: 3,8; 5,7; 6,9; 8,2; 8,9; 9,4; 10,0; 10,7; 11,3; 12,3; 13,9; 14,3: 14,6; 15,1; 15,8; 16,6; 17,0; 17,4; 18,0; 18,3; 18,9; 19,4; 19,7; 20,5; 20,8; 21,4; 22,0; 22,6; 23,6; 24,7; 25,3; 26,5; 27,4; 27,6; 28,2; 29,5; 30,7; 31,9; 32,5; 32,7; 33,0; 35,0; 35,8; 36,2 ± 0,2° 2Theta. Of those the most characteristic are the peaks at about: 6,9; 8,9; 15,1; 16,6; 17,4; 19,4; 19,7; 20,5; 20,8; 22,6; 23,6; 25,3°. Figure 3 shows typical x-ray powder diffraction pattern of candesartan cilexetil Form 6 with strongest peaks at about 6,9; 8,9; 16,6; 17,4; 19,4± 0,2°. The candesartan cilexetil Form 6 is further characterized by DSC, IR and m.p.

### A new crystalline form of candesartan cilexetil (Form 7)

To obtain a Form 7 of candesartan cilexetil is dissolved in chlorinated solvent, such as dichloromethane or chloroform in the concentration of 0,01 g-1.5 g of candesartan cilexetil /g of the solvent at room temperature and the obtained solution is concentrated in vacuum to the concentration of 0,55 g - 0,58 g of candesartan cilexetil / g of concentrate.and thus obtained concentrate is precipitated by quick addition (in few seconds) of a liquid straight chain hydrocarbon, preferably with n-heptane in the concentration of 2 ml - 50 ml of hydrocarbon / g of concentrate at room temperature, preferably about 5.7 ml of hydrocarbon/g of concentrate. A solid is formed which is a new crystalline form of candesartan cilexetil (Form 7) is for example characterized by an x-ray powder diffraction pattern having peaks at about 6,0; 9,0; 11,7; 12,0; 15,1; 16,3; 17,3; 19,4; 19,8; 21,0; 21,3; 22,0; 22,4; 23,3; 24,3; 25,3; 25,6; 26,0; 29,1 ± 0,2° 2Theta. The X-ray spectra can exhibit also the peaks at about 8,5; 10,2; 12,7; 14,4; 15,8; 17,9; 18,7; 26,6; 27,8; 28,4; 28,9; 29,5; 30,2; 31,3; 32,9; 33,4; 34,2; 35,7± 0,2° 2Theta. Figure 9 shows typical x-ray powder diffraction pattern of candesartan cilexetil Form 7 with strongest peaks at about 6,0; 9,0; 12,0; double peak between 19 and 20 (19,4 and 19,8); 21,3; 22,4; 25,6 ± 0,2° 2Theta °.

### A new crystalline form of candesartan cilexetil (Form 8)

To obtain a Form 8 of candesartan cilexetil, it is dissolved in chlorinated solvent, such as dichloromethane or chloroform in the concentration of 0,01g - 0,35 g of candesartan cilexetil / g of the solvent at room temperature and the obtained solution is concentrated in vacuum to the concentration of 0,40 g of candesartan cilexetil / g of concentrate and thus obtained concentrate is precipitated by quick addition(in few seconds) of a liquid straight chain hydrocarbon, preferably n-heptane or n-hexane in the concentration 2 ml-50 ml of hydrocarbon / g of the concentrate at room temperature, preferably about 8 ml of hydrocarbon / g of the concentrate a solid is formed which is a new crystalline form of Candesartan cilexetil (Form 8). The candesartan cilexetil Form 8 is characterized by an x-ray powder diffraction pattern having peaks at about 6,7; 7,1; 8,4; 9,7; 11,0; 11,9; 14,2; 14,5; 15,4; 16,3; 16,9; 17,8; 19,5; 20,2; 20,7; 21,6; 22,1; 23,1; 24,0; 24,6; 25,1; 25,5; 26,5; 26,9; 27,8; 28,6; 28,8; 30,7; 33,0 ± 0,2° 2Theta. Figure 10 shows typical x-ray powder diffraction pattern of candesartan cilexetil Form 8 with strongest peaks at about 7,1; 16,3; 17,8; 19,5; 20,2; 24,0 ± 0,2° 2Theta °.

### Amorphous candesartan cilexetil

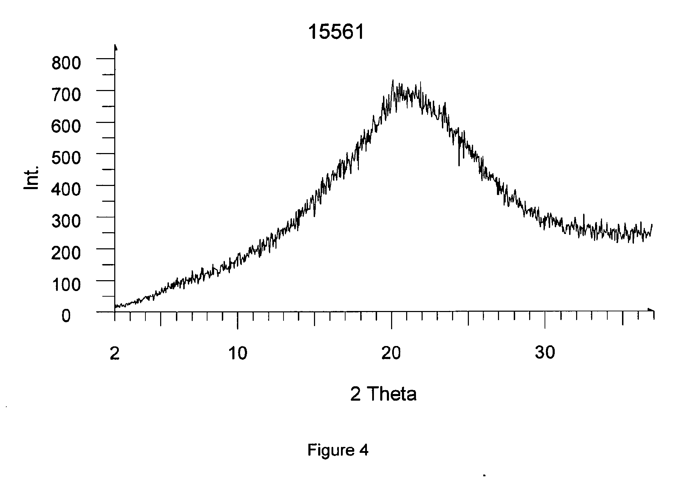

Candesartan cilexetil is dissolved in chlorinated solvent, preferably in dichloromethane or chloroform in the concentration of 0,01 g-0, 5 g of candesartan cilexetil/g of the solvent at room temperature and the obtained solution is concentrated in vacuum to the concentration of 0,3 g - 0,5 g of candesartan cilexetil/g of the concentrate and thus obtained concentrate is precipitated by quick addition (in few seconds) of a liquid cyclic hydrocarbon, preferably with cyclohexane or cyclopentane in the concentration of 10 ml - 40 ml of hydrocarbon/g of the concentrate at room temperature an amorphous solid is formed that exhibits a continuum of X-ray diffractions throughout the entire difractogram scale as presented on Figure 4 . Amorphous candesartan cilexetil obtainable in accordance with our process is further characterized by DSC; IR; m.p..

The solid dosage forms comprising the candesartan cilexetil Form 5 or Form 6 or Form 7 or Form 8 or amorphous candesartan cilexetil can be prepared by conventional method. Tablet can be for example manufactured by direct compression though wet granulation is another commonly used technique. In wet granulation at least one of the ingredients can be mixed or contacted with liquid and further processed to provide aggregates, the liquid can be partially or completely removed and optionally other or more of the same ingredients may be further added and solid dosage forms manufactured.

Tableting compositions may have in addition to active pharmaceutical ingredient few or many components depending upon the tableting method used, the release rate desired and other factors. For example, compositions of the present invention may contain inactive ingredients (excipients) which function as such as different fillers, binders, disintegrants, glidants, lubricants and excipients that enhance the absorption of drugs from gastrointestinal tract.

Suitable fillers may be selected from microcrystalline cellulose, powdered cellulose, lactose, starch, pregelatinized starch, sucrose, glucose, mannitol, sorbitol, calcium phosphate, calcium hydrogen phosphate, aluminium silicate, sodium chloride, potassium chloride, calcium carbonate, calcium sulphate, dextrates, dextrin, maltodextrin, glycerol palmitostearate, hydrogenated vegetable oil, kaolin, magenesium carbonate, magnesium oxide, polymethacrylates, talc, and others. Preferred fillers are microcrystalline cellulose and lactose. Suitable binders may be starch, pregelatinized starch, gelatine, sodium carboxymethylcellulose, polyvinylpyrrolidone, alginic acid, sodium alginate, acacia, carbomer, dextrin, ehylcellulose, guar gum, hydrogenated vegetable oil, methylcellulose, hydroxyethylcellulose, hydroxypropylcellulose, hydroxypropylmethylcellulose, glucose syrup, magnesium aluminium silicate, maltodextrin, polymethacrylates, zein. Preferably hydroxypropyl cellulose, hydroxypropyl methylcellulose and polyvinylpyrrolidone are used. Suitable disintegrants may be selected from starch, pregelatinized starch, sodium starch glycolate, sodium carboxymethylcellulose, cross-linked sodium carboxymrethylcellulose, calcium carboxymethylcellulose, methylcellulose, microcrystalline cellulose, powdered cellulose, polacrilin potassium, cross-linked polivinylpyrrolidone, alginic acid, sodium alginate, colloidal silicon dioxide, guar gum, magnesium aluminium silicate, and others. Preferred disintegrants are sodium starch glycolate, cross-linked carboxymethylcellulose sodium and cross-linked polyvinylpyrrolidone. Suitable glidants may be magnesium stearate, calcium stearate, aluminium stearate, stearic acid, palmitic acid, cetanol, stearol, polyethylene glycols of different molecular weights, magnesium trisilicate, calcium phosphate, colloidal silicon dioxide, talc, powdered cellulose, starch and others. Preferred glidant is colloidal silicilon dioxide. Suitable lubricants may be selected from stearic acid, calcium, magnesium, zinc or aluminium stearate, siliconized talc, glycerol monostearate, glycerol palmitostearate, hydrogenated castor oil, hydrogenated vegetable oil, mineral oil, light mineral oil, polyethylene glycol, sodium benzoate, sodium lauryl sulphate, sodium stearyl fumarate, talc and others. Preferred lubricants are calcium or magnesium stearate and stearic acid. Suitable absorption enhancers may be selected from surface active agents, fatty acids, middle chain glycerides, steroide detergents (salts of bile salts), acyl carnitine and alcanoloil choline (esters of carnitine and choline and fatty acids with middle chain and long chain), N-acyl derivatrives of alpha-amino acids and N-acyl derivatives of non-alpha-amino acids, chitosanes and other mucoadhesive polymers. Especially suitable absorption enhancers are sodium deoxycholate, sodium taurocholate, polisorbate 80, sodium lauryl sulfate, sodium dodecylsulfate, octanoic acid, sodium docusate, sodium laurate, glyceride monolaurate, stearic acid, palmitinic acid, palmitooleinic acid, glycerilmonooleate, sodium taurocholate, ethylenediaminetetraacetic acid, sodium edentate, sodium citrate, b-cyclodextrine and sodium salicylate.

Different salts or esters and different polymorph forms sometimes require different techniques Pharmaceutical composition comprising novel forms of candesartan cilexetil, incorporated into a pharmaceutically acceptable carrier, which may comprise above excipients can be prepared by suitable procedures for example by dry granulation or peletization.

In one embodiment of the invention one can prepare film coated tablets by direct compression. Amorphous candesartan Cilexetil or candesartan Cilexetil of Form 5 or Form 6 or Form 7 or Form 8 is mixed with lactose, microcrystalline cellulose, starch and mixture is sieved. A suitable glidang and/or lubricant is added and mixed again. Cores are tableted and coated with suitable suspension, for example comprising cellulose derivatives and titan dioxide in water or alcohol and the film coated tablets are polished with talc.

### EXPERIMENTAL PART

Infrared spectra were obtained with Nicolet Nexus FTIR spectrophotometer. Samples were analyzed in KBr and scanned from 400 to 4000 cm⁻¹ with 16 scans and 2 cm⁻¹ resolution. Table bellow depicts the differences of IR spectra of polymorph forms Form 5, Form 6 and amorphous form obtained according to our invention of candesartan cilexetil.

| Position of band (cm⁻¹) | | |
|---|---|---|
| **Form 5** 1554/1 | **Form 6** 418-1 | **amorphous** 1556/1 |
| 1756 | 1751 | 1754 |
| 1574 | - | - |
| 1465 | - | - |
| - | 1322 | 1322 |
| 1003 | - | - |
| 988 | 994 | 991 |
| 912 | - | - |
| - | 744 | - |
| | | |
| 751 | - | 751 |
| | | Lost of some bands |

Thermograms were obtained with Mettler Toledo DSC822^{e} differential scanning calorimeter. The sample (4-6 mg) was placed in an unsealed aluminium pan with one hole and heated at 5°C /min in the temperature range from 30°C to 200°C in the nitrogen (100 ml/min).

Powder X-ray diffraction spectra of the samples were recorded on Philips PW1710 with reflexion technique: CuKα radiation, range from 2° to 37° 2Theta, step 0.04° 2Theta, integration time 1 sec.

From an x-ray diffraction pattern of a powdery substance one can establish differences among different crystal lattices, and can obtain information on level of order i.e. crystallinity where lover crystallinity causes peaks to broaden. The ultimate form of non orderness of a solid is amorphous state which does not show the repeatability of molecular directions and positions in a solid. Completely amorphous substance thus shows diffuse dispersion of a roentgen radiation, which exhibits a continuum of diffractions throughout of whole the measured range.
The diffraction values for a crystalline substance will be substantially independent of the difractometer used, if the difractometer is calibrated the values can differ for about 0,05° 2 Theta, taking into account the rounding the differences in values lay in the order of ± 0,1° 2Theta, however the different recording conditions or differences in preparing or handling samples can cause the variations from the values reported for as much as ± 0,2 2Theta. The intensities of each specific diffraction peak are a function of various factors, one of those being a particle size and preferred orientation.

Skilled person will differentiate the forms which are the embodiment of our invention from other forms by comparing the whole x-ray powder diffraction patterns and specifically the strongest peaks or any three to five or more distinct peaks selected from the above listed peaks for each specific crystalline form.

In order to prepare a pharmaceutical composition physical properties were measured. Table below gives comparable solubility of Form 1 and Form 5 prepared in accordance with our invention.

| **Sample / Phosphate buffer** | | **C**_{**30**} **min (µg/ml)** | **C**_{**60**} **min (µg/ml)** |
|---|---|---|---|
| Form 1 | pH=6.76 | <0.2 | <0.2 |
| | pH=6.76 ( Physiologically relevant medium) | 2-57 - 3.26 | 2,63 - 3.47 |
| | pH=6.76 | <0.2 | <0.2 |
| Form 5 | pH=6.76 (Physiologically relevant medium) | 37.14 | 68.46 |

Physiologically relevant medium is a phosphate buffer at pH=6.76 + Sodiumtauroholat (2,5 mM) + Lecitin (0,5 mM). Surface area of Form 1 was 2.15 - 2,68 m²/g whereas the surface area of Form 5 was 7,66 m²/g (BET - isotherm), the hygroscopity as established by DVS - first cycle at 90 % humidity was 0,23% for Form I and 0,44% for Form 5.

Following examples further illustrate the invention, They are provided for illustrative purposes only and are not intended to limit in any way the invention.

### EXPERIMENT 1 (candesartan cilexetil Form 5)

0,5g of candesartan cilexetil was dissolved in 15 ml of dichloromethane. The solution was evaporated in vacuum to the rest of 1,9 g, then 20 ml of methylcyclohexane was added under stirring in one portion at room temperature. Gummy precipitate was obtained, which crystallized overnight. Product was filtered and vacuum dried at 50°C.Yield:0,43g. m.p.:118,2°C-123,3°C

### EXPERIMENT 2 (candesartan cilexetil Form 5)

1g of candesartan cilexetil was dissolved in 30 ml of dichloromethane.The solution was evaporated in vacuum to the rest of 3,79 g, then 10 ml of n-heptane was added under stirring in one portion at room temperature. Gummy precipitate was obtained, which crystallized overnight. Product was filtered and vacuum dried at 50°C. Yield:1,03 g. m.p.:112.9°C-117,0°C

### EXPERIMENT 3 (candesartan cilexetil Form 5)

6g of candesartan cilexetil was disolved in cca 13 ml of dichloromethane.Clear solution (22,8 g) was seeded with candesartan cilexetil Form 5,and 60 ml of n-heptane was added under stirring at room temperature. Gummy precipitate was obtained, which crystallized overnight. Product was filtered and dried at 50°C. Yield: 6,19g Form 5

### EXPERIMENT 4 (candesartan cilexetil amorphous)

0,5g of candesartan cilexetil was dissolved in 15 ml of dichloromethane or in chloroform. The solution was evaporated in vacuum to the rest of 1,25g, then 10 ml of cyclohexane or cyclopentane was added in one portion. Gummy precipitate was obtained, which crystallized in 1-2 hours. White suspension was stirred another hour at 5-10°C. Product was filtered and vacuum dried at 50°C.Yield:0,44g Amorphous.. m.p.:106,0°C-109,4°C

### EXPERIMENT 5 (candesartan cilexetil Form 6)

1g of candesartan cilexetil was dissolved in 15 ml of dichloromethane.The solution was carefully evaporated in vacuum to the rest of 5,66 g (less than in experiments 1 to 3). 40 ml of MCH was added under stirring at room temperature and stirring was continued overnight. Product was filtered and vacuum dried at 50°C.Yield: 0,93 g . Form 6 m.p.:121,3°C-127,5°C.

### EXPERIMENT 6 (Candersartan cilexetil Form 6)

1g of candesartan cilexetil was dissolved in 30 ml of dichloromethane.The solution was evaporated in vacuum to the rest of 1,67g (more than in experiments 1 to 4). Oily residue was seeded with candesartan cilexetil Form 6 and 40 ml of MCH was added under stirring at room temperature. Stirring was continued overnight. Product was filtered and vacuum dried at 50°C. Yield:0,87g. Form 6

### EXPERIMENT 7 (Candersartan cilexetil Form 5)

A solution of 300 ml of methanol and 7,6 ml of methanesulfonic acid is cooled to approximately 3°C. 50 g of trityl candesartan cilexetil is added and the mixture is stirred at approximately 3°C for 30 minutes. 24.4 ml of triethylamine are added at a rate that temperature does not exceed 10°C. 1000 ml of heptane are added followed by 50 ml of H2O. The mixture is stirred for 10 min in an ice bath. The layers are then separated, the lower phase is extracted with 250 ml of heptane under ice cooling. The layers are again separated and the lower phase is warmed up to approximately 20°C. 1 5 ml of 2 m HCl is added drop wise followed by seeds of candesartan cilexetil. The suspension is briefly stirred and 14.3 ml of 2 m HCl are added within approximately 60 min. The suspension is then stirred for additional 30 min and the product is then isolated by filtration, washed with 75 ml of a mixture methanol and H2O ( 3:1 v/v) and dried in vacuum at ambient temperature over night.

### EXPERIMENT 8 (Candesartan cilexetil Form 7)

1g of Candesartan cilexetil was dissolved in 30 ml of dichloromethane.The solution was evaporated in vacuum to the rest of 1,76g. 10 ml of n-heptane was added under stirring at room temperature. Stirring was continued overnight. Product was filtered and vacuum dried at 50°C.Yield:0,08g. Form 7

### EXPERIMENT 9 (Candesartan cilexetil Form 8)

1g of Candesartan cilexetil was dissolved in 30 ml of dichloromethane.The solution was evaporated in vacuum to the rest of 2,53 g. 20 ml of n-heptane was added under stirring at room temperature. Stirring was continued overnight. Product was filtered and vacuum dried at 50°C. Yield:0,98g. Form 8

## Claims

1. A process for preparing polymorph forms of candesartan cilexetil selected form the group of Form 5, Form 6, Form 7, Form 8 or amorphous candesartan **characterized in that** each of the polymorph form is obtained by a process which comprises:
a) dissolving candesartan cilexetil in chlorinated solvent;
b) concentrating thus obtained solution;
c) adding a liquid hydrocarbon to the above concentrated solution;
**characterized in that**
i) if the aforesaid solution is concentrated until concentration of about 0,26 g to about 0,27 g of candesartan cilexetil / g of the concentrate a Form 5 is obtained and
ii) if the aforesaid solution is concentrated until concentration of about 0,15 g to about 0,21 g or alternatively of about 0,6 g to about 1,5 g of candesartan cilexetil / g of the concentrate a Form 6 is obtained and
iii) if the aforesaid solution is concentrated until concentration of about 0,55 g to about 0,55 g of candesartan cilexetil / g of the concentrate a Form 7 is obtained and
iv) if the aforesaid solution is concentrated until concentration around 0,4 g of candesartan cilexetil / g of the concentrate a Form 8 is obtained and further **characterized in that** if the aforesaid solution is concentrated until concentration of about 0,3 g to about 0,4 g of candesartan cilexetil / g of the concentrate amorphous candesartan cilexetil is obtained after addition of a liquid hydrocarbon selected from cycloalkanes.

2. A polymorph form of candesartan cilexetil selected form the group of Form 5, Form 6, Form 7, Form 8 or amorphous candesartan **characterized in that** it is obtained by a process which comprises:
a) dissolving candesartan cilexetil in chlorinated solvent;
b) concentrating thus obtained solution;
c) adding a liquid hydrocarbon to the above concentrated solution; **characterized in that** if the aforesaid solution is concentrated until concentration of about 0,26 g to about 0,27 g of candesartan cilexetil / g of the concentrate a Form 5 is obtained and if the aforesaid solution is concentrated until concentration of about 0,15 g to about 0,21 g or alternatively of about 0,6 g to about 1,5 g of candesartan cilexetil / g of the concentrate a Form 6 is obtained and if the aforesaid solution is concentrated until concentration of about 0,55 g to about 0,55 g of candesartan cilexetil / g of the concentrate a Form 7 is obtained and if the aforesaid solution is concentrated until concentration around 0,4 g of candesartan cilexetil / g of the concentrate a Form 8 is obtained and further **characterized in that** if the aforesaid solution is concentrated until concentration of about 0,3 g to about 0,4 g of candesartan cilexetil / g of the concentrate amorphous candesartan cilexetil is obtained after addition of a liquid hydrocarbon selected from cycloalkanes.

3. A process for the preparation of crystalline candesartan cilexetil of Form 5, which comprises:
a) dissolving candesartan cilexetil in chlorinated solvent;
b) concentrating thus obtained solution until concentration of about 0,26 g to about 0,27 g of candesartan cilexetil / g of the concentrate;
c) adding a liquid hydrocarbon to the above concentrated solution;
d) isolating formed crystals of crystalline candesartan cilexetil of Form 5.

4. A process for the preparation of crystalline candesartan cilexetil of Form 6, which comprises:
a) dissolving candesartan cilexetil in chlorinated solvent;
b)) concentrating thus obtained solution until concentration of about 0,15 g to about 0,21 g or alternatively of about 0,5 g to about 1,5 g of candesartan cilexetil / g of the concentrate;
c) adding a liquid hydrocarbon to the above concentrated solution;
d) isolating formed crystals of crystalline candesartan cilexetil of Form 6.

5. A process for the preparation of amorphous candesartan cilexetil, which comprises:
a) dissolving candesartan cilexetil in a chlorinated solvent;
b) concentrating thus obtained solution until concentration of about 0,3 g to about 0,5 g of candesartan cilexetil / g of the concentrate;
c) precipitating the amorphous candesartan cilexetil by adding a liquid cyclic hydrocarbon to the above concentrated solution.

6. The crystalline candesartan cilexetil Form 5, **characterized by** an x-ray powder diffraction pattern comprising peaks at about 6,1; 11,6; 20,0; 21,2; 25,6 ± 0,2° 2Theta.

7. The crystalline candesartan cilexetil Form 5, **characterized by** an x-ray powder diffraction pattern substantially as depicted on Figure 1.

8. The crystalline candesartan cilexetil Form 6, **characterized by** an x-ray powder diffraction pattern comprising peaks at about 6,9; 8,9; 16,6; 17,4; 19,4t 0,2° 2Theta.

9. The crystalline candesartan cilexetil Form 6, **characterized by** an x-ray powder diffraction pattern substantially as depicted on Figure 3.

10. The crystalline candesartan cilexetil Form 7, **characterized by** an x-ray powder diffraction pattern comprising peaks at about 7,1; 16,3; 17,8; 19,5; 20,2; 24,0 ± 0,2° 2Theta.

11. The crystalline candesartan cilexetil Form 7, **characterized by** an x-ray powder diffraction pattern substantially as depicted on Figure 9.

12. The crystalline candesartan cilexetil Form 8, **characterized by** an x-ray powder diffraction pattern comprising peaks at about 6,9; 8,9; 16,6; 17,4; 19,4± 0,2° 2Theta.

13. The crystalline candesartan cilexetil Form 8, **characterized by** an x-ray powder diffraction pattern substantially as depicted on Figure 10.

14. An amorphous candesartan cilexetil, **characterized by** an x-ray powder diffraction pattern exhibiting a continuum of diffractions substantially throughout the measured range from 2° to 37° 2Theta prepared by a process which comprises precipitating the amorphous candesartan cilexetil by adding a liquid cyclic hydrocarbon to the concentrated solution in a chlorinated solvent.

15. The amorphous candesartan cilexetil, according to previous claim, **characterized by** an x-ray powder diffraction pattern substantially as depicted on Figure 4.

16. Use of candesartan cilexetil Form 5 or candesartan cilexetil Form 6 or candesartan cilexetil Form 7 or candesartan cilexetil Form 8 or amorphous candesartan cilexetil as a medicament.

17. Use of candesartan cilexetil Form 5 or candesartan cilexetil Form 6 or candesartan cilexetil Form 7 or candesartan cilexetil Form 8 or amorphous candesartan cilexetil for the preparation of a medicament for treating hypertensive diseases or hypercardia or heart failure or cardiac infarction or stroke or cerebral apoplexy or nephritis.

18. A pharmaceutical composition comprising candesartan cilexetil Form 5 or candesartan cilexetil Form 6 or candesartan cilexetil Form 7 or candesartan cilexetil Form 8 or amorphous candesartan cilexetil and a pharmaceutically acceptable carrier.
